# FASCICULE DE BREVET EUROPEEN

(11) **EP 3 875 136 B1**
(45) Date de publication et mention de la délivrance du brevet: **16.10.2024**
(21) Numéro de dépôt: 21151993.9
(22) Date de dépôt: 18.01.2021
(51) Int. Cl.: A61M 16/08, A61M 39/26, F16L 37/60, A61M 16/10

(54) **VENTILATEUR MÉDICAL COMPRENANT UNE PRISE DE RACCORDEMENT INCLUANT UN FILTRE AISÉMENT EXTRACTIBLE**
MEDIZINISCHES VENTILATIONSGERÄT MIT EINEM ANSCHLUSSSTUTZEN, DER EINEN LEICHT ENTFERNBAREN FILTER ENTHÄLT
MEDICAL VENTILATOR WITH A CONNECTION SOCKET INCLUDING AN EASILY REMOVABLE FILTER

(30) Priorité: 03.03.2020 FR 2002149
(43) Date de publication de la demande: 08.09.2021
(73) Titulaire: Air Liquide Medical Systems, 92160 Antony (FR)
(72) Inventeur: LOPEZ, Julien, 92160 Antony (FR); GIARD, Pauline, 92160 Antony (FR); RUDNIANYN, Philippe, 92160 Antony (FR)
(74) Mandataire: Air Liquide

(56) Documents cités:
- EP-A1- 1 611 921
- DE-U1- 202013 008 155
- FR-A1- 2 431 649

## Description

L'invention concerne un ventilateur médical équipé d'une prise de raccordement de raccordement permettant d'alimenter le ventilateur en un gaz médical, typiquement de l'oxygène, en particulier une prise de type NIST ou DISS.

Dans les hôpitaux, les réseaux de distribution de fluides, c'est-à-dire les canalisations acheminant les fluides médicaux, telles gaz ou le vide (i.e. dépression/aspiration), se terminent par des prises murales fixées sur les parois des bâtiments auxquelles peuvent être raccordés des appareils médicaux consommant du gaz ou nécessitant du vide pour fonctionner. A ce titre, on peut citer les prises dites « BM ».

Ces prises murales ont une structure particulière comme enseigné par EP-A-1611921 et FR-A-7821370. Ces prises ne sont pas utilisées dans les ventilateurs médicaux car elles sont conçues pour être agencées directement sur les parois ou dans des caissons eux-mêmes fixées aux parois des bâtiments, en étant alimentées par les canalisations de fluide qui parcourent les bâtiments hospitaliers.

Parmi les prises de raccordement pour ventilateurs médicaux, c'est-à-dire celles qui sont intégrées aux ventilateurs, les plus utilisées pour fournir des gaz, sont les prises de type NIST (Non Interchangeable Screw-Threaded Connecter) et de type DISS (Diameter Index Safety System ). Ces prises de raccordement sont montées directement sur les appareils d'assistance respiratoire, c'est-à-dire les ventilateurs médicaux, servant à fournir un gaz respiratoire, par exemple de l'air ou de l'air enrichi en oxygène, à des patients souffrant de troubles respiratoires.

La prise de raccordement d'un ventilateur médical est en communication fluidique avec le circuit ou chemin de gaz interne du ventilateur médical et assure par ailleurs le raccordement d'une source de gaz médical extérieure alimentant le ventilateur médical. Ainsi, elle peut être alimentée avec de l'oxygène de manière à pouvoir enrichir en oxygène, l'air fourni par la micro-soufflante du ventilateur médical au patient, c'est-à-dire de produire un flux d'air enrichi en oxygène (i.e. teneur en O₂ > 21% vol.).

Ces prises de raccordement pour ventilateurs médicaux comprennent habituellement un corps de prise traversé axialement par un passage interne comprenant des moyens de contrôle du flux de gaz dans le passage interne, à savoir en général un clapet mobile coopérant avec un siège de clapet, typiquement un clapet en silicone ou analogue.

Un problème connu est lié à la pollution interne de la prise et du circuit de gaz interne du ventilateur médical par des poussières ou d'autres polluants particulaires pouvant y pénétrer par la prise de raccordement, et aller ensuite détériorer le clapet et/ou empêcher qu'une bonne étanchéité fluidique ne puisse être assurée par celui-ci.

En effet, les sources de gaz ne sont pas toutes parfaitement propres et par ailleurs le stockage et/ou l'inutilisation des ventilateurs médicaux pendant des durées plus ou moins longues peuvent conduire à des entrées de poussières ou autres dans les prises de raccordement desdits ventilateurs médicaux.

Pour tenter d'y remédier, il a été proposé d'insérer un filtre dans une chambre aménagée dans le passage interne du corps de prise, en amont du clapet en silicone. Or, cette opération oblige un démontage de la prise de raccordement, en particulier une extraction du clapet et de son siège afin de pouvoir y insérer le filtre, puis un remontage de ces éléments. De telles opérations de montage/démontage de la prise et de ses éléments internes doivent être aussi répétés lors de chaque maintenance de la prise et/ou lors du remplacement du filtre lorsqu'il est encrassé, avec en plus une obligation de démonter à chaque fois, la prise du ventilateur médical sur lequel elle est fixée.

On comprend aisément que ceci n'est pas pratique et très fastidieux, que cela nécessite des interventions longues de techniciens de maintenance et engendre par ailleurs des risques de dégradation du ventilateur médical pendant sa maintenance, sans parler de l'immobilisation du ventilateur médical lui-même qui ne peut être utilisé pendant ces opérations de maintenance.

Au vu de cela, le problème qui se pose est de pouvoir simplifier et accélérer la maintenance des prises de raccordement des ventilateurs médicaux, de préférence les prises de type NIST ou DISS, en particulier de pouvoir remplacer un filtre sans avoir à ouvrir le ventilateur médical, à en extraire la prise de raccordement et à la démonter pour en extraire le filtre.

L'invention est défini par le jeu de revendications annexé .

La solution concerne alors un ventilateur médical comprenant un circuit de gaz interne en communication fluidique avec une prise de raccordement agencée sur ledit ventilateur médical, c'est-à-dire intégrée audit ventilateur, ladite prise de raccordement comprenant un corps de prise traversé axialement par un passage interne reliant un orifice d'entrée porté par une extrémité proximale libre du corps de prise à un orifice de sortie, et comprenant des moyens de contrôle du flux pour contrôler la circulation du gaz au sein dudit passage interne agencés dans le passage interne entre les orifices d'entrée et de sortie, et un élément de filtration agencé dans le passage interne du corps de prise.

Selon la technologie, l'élément de filtration est monté de manière extractible au sein du passage interne du corps de prise, l'insertion ou l'extraction, i.e. son montage/démontage, dudit élément de filtration se faisant par l'orifice d'entrée du passage interne du corps de prise porté par l'extrémité proximale libre du corps de prise, c'est-à-dire sans qu'il n'y ait besoin de démonter ou dissocier la prise et/ou d'ouvrir le ventilateur médical.

Selon le mode de réalisation considéré, le ventilateur médical peut comprendre l'une ou plusieurs des caractéristiques suivantes :
- l'orifice d'entrée du corps de prise est situé à l'extrémité proximale libre de la prise de raccordement destinée à venir se raccorder une source de gaz médical pour fournir du gaz médical à la prise, telle une source d'oxygène.
- l'orifice de sortie du corps de prise est situé à l'autre extrémité ou extrémité distale de la prise de raccordement destinée à venir se raccorder au circuit de gaz interne d'un ventilateur médical.
- le gaz entre dans le passage de gaz du corps de prise par l'orifice d'entrée et en ressort par l'orifice de sortie, c'est-à-dire dans le sens allant de l'orifice d'entrée à l'orifice de sortie.
- l'élément de filtration est agencé dans une cartouche à filtre.
- la cartouche à filtre comprend un corps de cartouche traversé axialement par un conduit interne contenant l'élément de filtration.
- la cartouche à filtre est montée coaxialement au passage interne du corps de prise.
- le corps de cartouche est conformé pour être fixé par vissage dans le passage interne du corps de prise.
- le passage interne du corps de prise comprend plusieurs sections internes de diamètres différents.
- le corps de cartouche comprend un filetage agencé dans sa paroi périphérique externe.
- au moins une partie du passage interne du corps de prise comprend un taraudage interne complémentaire du filetage porté par la paroi périphérique externe du corps de cartouche
- le corps de cartouche de la cartouche à filtre comprend une extrémité avant, aussi appelée « tête », conformée pour pouvoir être entrainée en rotation par un outil de vissage, lors du vissage ou du dévissage de ladite cartouche à filtre au sein du passage interne du corps de prise, en particulier l'extrémité avant du corps de cartouche comprend une empreinte pour un outil de vissage/dévissage.
- l'empreinte est de forme polygonale.
- Préférentiellement, l'empreinte est de forme hexagonale, c'est-à-dire configurée pour recevoir une clé à 6 pans ou analogue.
- le corps de cartouche a une forme générale tubulaire, de préférence présentant plusieurs sections de diamètres externes différents.
- le corps de cartouche est en laiton ou en tout autre métal ou alliage métallique.
- l'élément de filtration comprend un filtre fritté, de préférence en bronze.
- alternativement, l'élément de filtration comprend un filtre tissé, de préférence monté sur un support en polymère.
- l'élément de filtration comprend un filtre fritté présentant des « pores » de diamètres compris entre 60 et 100 µm environ.
- l'élément de filtration comprend un filtre de forme conique ou autre.
- les moyens de contrôle du flux comprennent un clapet mobile coopérant avec un siège de clapet.
- le clapet mobile est mobile axialement dans le passage interne du corps de prise.
- la cartouche à filtre est montée coaxialement au passage interne du corps de prise.
- le clapet mobile est en silicone.
- le clapet mobile a une forme de parapluie, c'est-à-dire qu'il comprend une tige surmontée d'un disque.
- la prise est choisie parmi les prises de type NIST ou DISS.
- le corps de prise est de forme allongée.
- le corps de prise est en laiton, de préférence chromé ou nickelé.
- le corps de prise est conformé pour y raccorder mécaniquement et fluidiquement une source de gaz, notamment d'oxygène, en particulier via une conduite d'amenée de gaz, telle un tuyau flexible ou analogue.
- la prise de raccordement est agencée de manière à alimenter le circuit de gaz interne en gaz, en particulier de l'oxygène.
- une source de gaz, en particulier une source d'oxygène, telle une bouteille d'oxygène, est reliée fluidiquement à la prise de raccordement du ventilateur pour lui fournir du gaz, en particulier de l'oxygène.
- une source de gaz est reliée fluidiquement à la prise de raccordement du ventilateur par l'intermédiaire d'une conduite de gaz flexible ou analogue munie d'un connecteur de raccordement complémentaire de la prise de raccordement.
- le circuit de gaz interne du ventilateur est par ailleurs relié fluidiquement à une micro-soufflante, aussi appelée turbine ou compresseur.
- il comprend une carcasse ou coque externe portant la prise de raccordement.
- il comprend en outre des moyens de pilotage à microprocesseur, telle une carte électronique comprenant un (ou des) microprocesseurs, notamment un microcontrôleur.
- les moyens de pilotage pilotent la micro-soufflante.
- il comprend en outre des moyens d'alimentation électrique, tels que batterie rechargeable et/ou raccordement au secteur (1 10/220V) avec préférentiellement un transformateur de courant et/ou de tension.

La technologie porte aussi sur un ensemble de ventilation d'un patient comprenant :
- un ventilateur médical comprenant une prise de raccordement selon l'invention,
- une source de gaz, en particulier d'oxygène, raccordée fluidiquement à la prise de raccordement du ventilateur médical, et
- une interface respiratoire patient reliée fluidiquement à une sortie de gaz du ventilateur médical par l'intermédiaire d'un tuyau d'amenée de gaz flexible.

L'invention va maintenant être mieux comprise grâce à la description détaillée suivante, faite à titre illustratif mais non limitatif, en référence aux figures annexées parmi lesquelles :
Fig. 1 est une vue de face d'une prise de type DISS pour un ventilateur médical selon l'invention,
Fig. 2 est une vue en coupe longitudinale d'une prise de type DISS pour un ventilateur médical selon l'invention, représentée portée par une pièce-support du ventilateur médical,
Fig. 3 est une vue de face d'une prise de type NIST pour un ventilateur médical selon l'invention,
Fig. 4 est une vue en coupe longitudinale d'une prise de type NIST pour un ventilateur médical selon l'invention, représentée portée par une pièce-support du ventilateur médical,
Fig. 5 est une vue en coupe longitudinale de la cartouche à filtre d'une prise pour un ventilateur médical selon l'invention, et
Fig. 6 schématise un ensemble de ventilation d'un patient comprenant un ventilateur médical équipé d'une prise selon l'invention.

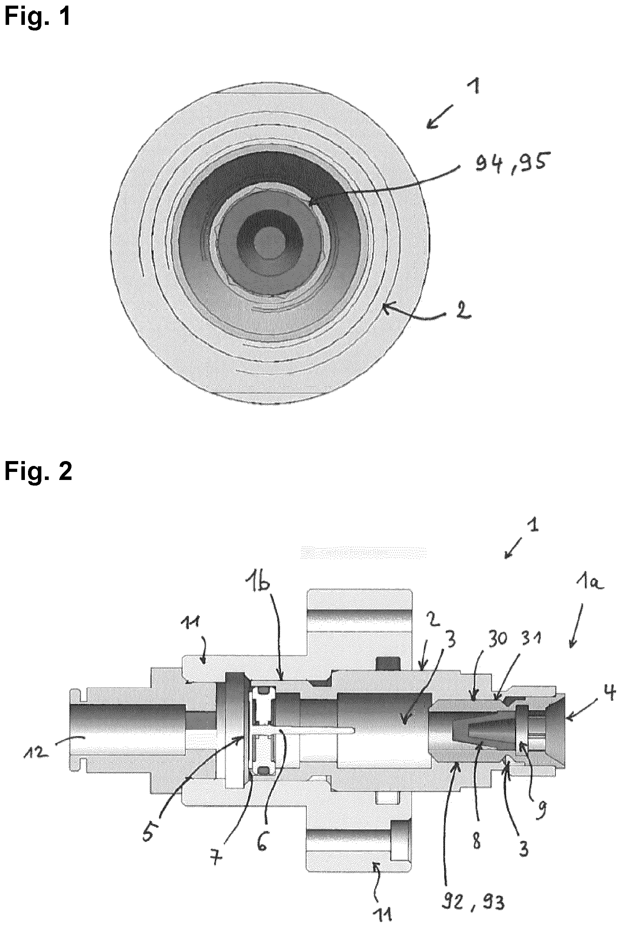

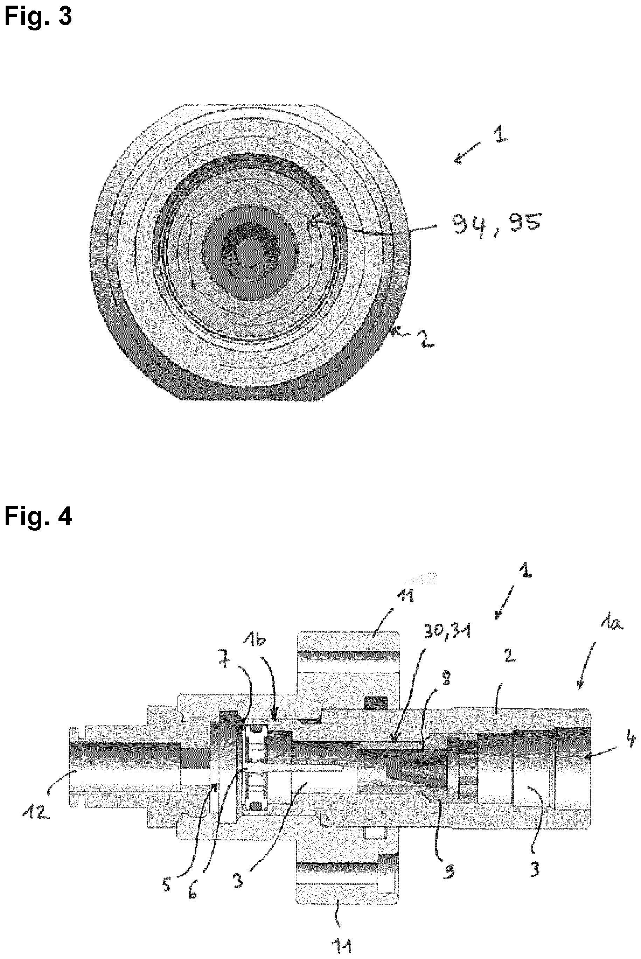

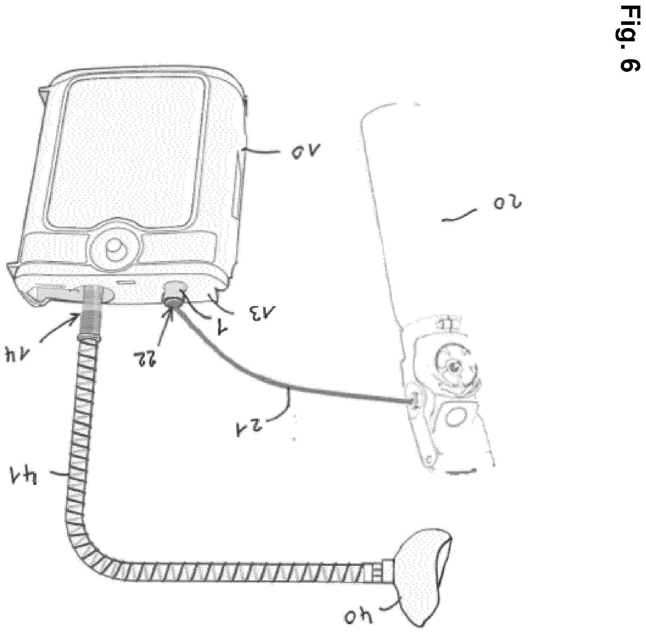

La Fig. 2 est une vue en coupe longitudinale d'un mode de réalisation d'une prise de raccordement 1 de type DISS pour un ventilateur médical 10 selon l'invention, et, de manière similaire, la Fig. 4 est une vue en coupe longitudinale d'un mode de réalisation d'une prise de raccordement 1 de type NIST pour un ventilateur médical 10 selon l'invention, lesquelles prises 1 des Fig. 2 et Fig. 4 sont représentées portées par une pièce-support 11 fixée à la carcasse 13 dudit ventilateur médical 10 (cf. Fig. 6), par exemple par des vis ou analogues , et par ailleurs reliées fluidiquement, via leur extrémité distale 1b, au circuit de gaz interne 12 du ventilateur médical 10.

Comme on le voit, la structure et le fonctionnement des prises de raccordement 1 de type DISS et NIST des Fig. 2 et Fig. 4 sont (quasi) identiques. Plus précisément, la prise de raccordement 1 pour ventilateur médical 10, qu'elle soit de type de type DISS ou NIST, comprend un corps de prise 2 de forme allongée, i.e. oblongue, traversé axialement par un passage interne 3 reliant un orifice d'entrée 4 de gaz à un orifice de sortie 5 de gaz.

Le gaz, par exemple de l'oxygène, entre dans le passage de gaz 3 par l'orifice d'entrée 4 qui est porté par l'extrémité proximale 1a libre de la prise 1, et en ressort par l'orifice de sortie 5 qui est porté par l'extrémité distale 1b de la prise 1, c'est-à-dire qu'il y circule dans le sens allant de l'orifice d'entrée 4 à l'orifice de sortie 5.

Le corps de prise 2 est par exemple en laiton nickelé et est par ailleurs conformée pour que l'on puisse y raccorder mécaniquement et fluidiquement une source de gaz 20, notamment d'oxygène, via une conduite d'alimentation en gaz, tel un tuyau flexible ou analogue, munie d'un connecteur 22 de forme et/ou structure complémentaires, comme illustré en Fig. 6, venant se raccorder à l'extrémité proximale libre 1a de la prise 1.

En outre, sont prévus des moyens de contrôle du flux 6, 7 pour contrôler la circulation du gaz au sein du passage interne 3, lesquels sont agencés dans le passage interne 3 entre les orifices d'entrée 4 et de sortie 5. Typiquement, les moyens de contrôle du flux 6, 7 comprennent un clapet mobile 6 coopérant avec un siège de clapet 7, en particulier un clapet en silicone ayant une forme de parapluie, c'est-à-dire formé d'une tige surmontée d'un disque plat ou légèrement bombé.

Afin de garantir une bonne hygiène de la prise, c'est-à-dire d'empêcher que des poussières ou autres polluants particulaires ne puissent y pénétrer et aller ensuite polluer le circuit de gaz interne 12 du ventilateur médical 10, un élément de filtration 8 est agencé dans le passage interne 3 du corps de prise 2.

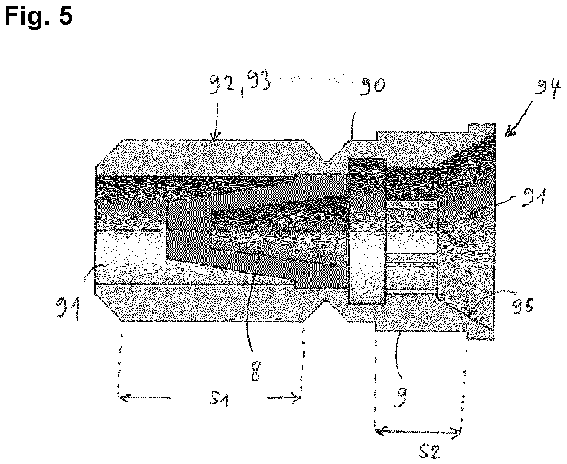

L'élément de filtration 8 peut être inséré et fixé directement dans le passage 3 ou préférentiellement, comme dans les modes de réalisation proposés, indirectement en étant inséré dans une cartouche à filtre 9 comprenant un corps de cartouche 90 traversé axialement par un conduit interne 91 au sein duquel est disposé l'élément de filtration 8, comme détaillé sur la Fig. 5.

Dans tous les cas, selon l'invention, l'élément de filtration 8 est monté de manière extractible au sein du passage interne 3 du corps de prise 2, depuis l'extrémité proximale 1a de la prise 1 au travers de l'orifice d'entrée 4 du corps de prise 2, c'est-à-dire que l'insertion ou l'extraction dudit élément de filtration 8 se fait par l'extrémité proximale 1a et l'orifice d'entrée 4 du passage interne 3 du corps de prise 2 sans qu'il ne soit nécessaire de dissocier la prise 1 du ventilateur 10 et/ou de démonter la prise 1 elle-même pour y insérer ou en extraire l'élément de filtration 8.

Dans ce mode de réalisation, l'élément de filtration 8 est un filtre de type fritté, par exemple en bronze, de forme ici conique et présentant des pores de diamètre suffisamment petits pour retenir les polluants particulaires solides et autres poussières, tout en laissant passer le gaz qui alimente la prise 1, par exemple de l'oxygène devant être fourni au ventilateur 10 par une source d'oxygène, telle une bouteille d'oxygène. Par exemple, on peut utiliser le filtre fritté en bronze avec pores de 50 à 100 µm environ. Bien entendu, un autre type de filtre peut être utilisé.

Comme visible sur la Fig. 5, afin de permettre sa fixation par vissage dans le passage interne 3, le corps de cartouche 90 est conformé de manière à présenter un filetage 92 sur sa paroi périphérique externe 93, lequel est complémentaire d'un taraudage 30 aménagé dans la région d'entrée 31 du passage 3 du corps de prise 2 de manière à permettre une fixation par vissage de la cartouche à filtre 9 au sein du passage 3 du corps de prise 2 comme illustré sur les Fig. 2 et Fig. 4.

Afin de faciliter son montage par vissage et son démontage par dévissage, le corps de cartouche 90 de la cartouche à filtre 9 comprend aussi une extrémité avant 94 particulière, à savoir qu'elle est conformée pour pouvoir être entrainée en rotation par un outil de vissage, lors du vissage ou du dévissage de la cartouche à filtre 9 au sein du passage interne 3 du corps de prise 2, via l'extrémité proximale 1a de la prise 1, comme visible sur les Fig. 1 et Fig. 3. Par exemple, l'extrémité avant 94 du corps de cartouche 90 présente une empreinte 95 de forme hexagonale pouvant coopérer avec une clé à 6 pans.

Dans les modes de réalisation présentés, le corps de cartouche 90 a une forme générale tubulaire comprenant plusieurs sections successives de différents diamètres externes, à savoir ici une section avant S1 de diamètre inférieur à celui de la section arrière S2, comme illustré sur la Fig. 5. Il peut être réalisé en laiton ou un autre métal ou alliage métallique.

La Fig. 6 schématise un ensemble de ventilation d'un patient incluant un ventilateur médical 10, c'est-à-dire un appareil d'assistance respiratoire, comprenant une carcasse ou coque externe 13 portant la prise de raccordement 1, c'est-à-dire que la prise 1 conforme à l'invention, laquelle est fixée à la carcasse 13 par l'intermédiaire par exemple de la pièce-support 11 visible sur les Fig. 2 et Fig. 4. Autrement dit, la prise 1 de l'invention fait saillie vers l'extérieur en éloignement par rapport à la carcasse 13 du ventilateur 10.

La prise de raccordement 1 selon l'invention est avantageusement une prise de type NIST ou DISS. Lorsque la prise 1 est de type DISS, on veille à ce qu'elle réponde aux exigences géométriques définies par la norme CGA V-5-2008, et aux exigences géométriques de la norme NF EN ISO 5359 lorsque la prise 1 est de type NIST.

Comme déjà expliqué, la prise de raccordement 1 équipant un ventilateur 10 de l'invention est en communication fluidique, d'une part, via son extrémité proximale 1a, avec une source de gaz médical 20, typiquement une source d'oxygène, par exemple une bouteille d'oxygène comprimé, fournissant le gaz médical à l'orifice d'entrée 4 de la prise 1 et, d'autre part, avec le circuit de gaz interne 12 du ventilateur médical 10 afin de fournir le gaz médical, typiquement de l'oxygène, audit circuit 12 au travers de l'orifice de sortie 5 porté par l'extrémité distale 1b de la prise 1. Ceci permet par exemple d'enrichir en oxygène, le flux d'air délivré par la micro-soufflante, aussi appelée turbine ou compresseur, du ventilateur 10 et de fournir de l'air enrichi en oxygène (i.e. teneur en O₂ > 21% vol.) à un patient.

La source de gaz médical 20 est reliée au ventilateur 10 via une conduite de gaz flexible 21 ou analogue munie d'un connecteur de raccordement 22 complémentaire de la prise de raccordement 1, par exemple formant ensemble une connexion de type mâle/femelle.

Par ailleurs, le ventilateur 10 comprend aussi une sortie de gaz 14 qui est reliée fluidiquement à une interface respiratoire patient 40, tel un masque respiratoire ou analogue, par l'intermédiaire d'un tuyau d'amenée de gaz 41 flexible.

Le ventilateur 10 est utilisable non seulement en milieu hospitalier mais aussi sur le terrain, c'est-à-dire dans un véhicule d'urgence type SAMU, SMUR ou analogue.

## Revendications

1. Ventilateur médical (10) comprenant un circuit de gaz interne (12) en communication fluidique avec une prise de raccordement (1) agencée sur ledit ventilateur médical (10), ladite prise de raccordement (1) comprenant :
- un corps de prise (2) traversé axialement par un passage interne (3) reliant un orifice d'entrée (4) porté par une extrémité proximale libre (1a) du corps de prise (2) à un orifice de sortie (5), et comprenant des moyens de contrôle du flux (6, 7) pour contrôler la circulation du gaz au sein dudit passage interne (3) agencés dans le passage interne (3) entre les orifices d'entrée (4) et de sortie (5), lesdits moyens de contrôle du flux (6, 7) comprenant un clapet mobile (6) coopérant avec un siège de clapet (7) et
- un élément de filtration (8) agencé dans le passage interne (3) du corps de prise (2), l'élément de filtration (8) étant monté de manière extractible au sein du passage interne (3) du corps de prise (2), l'insertion ou l'extraction dudit élément de filtration (8) se faisant par l'orifice d'entrée (4) du passage interne (3) porté par l'extrémité proximale libre (1a) du corps de prise (2), ledit élément de filtration (8) étant agencé dans une cartouche à filtre (9) comprenant un corps de cartouche (90) traversé axialement par un conduit interne (91), agencé dans le conduit interne (91),
**caractérisé en ce que** :
- le clapet mobile (6) est en silicone et a une forme de parapluie formé d'une tige surmontée d'un disque plat ou légèrement bombé, et
- l'élément de filtration (8) est un filtre fritté ou tissé.

2. Ventilateur selon la revendication 1, **caractérisé en ce que** le corps de cartouche (90) à filtre (9) comprend un filetage (92) agencé dans sa paroi périphérique externe (93) pour être fixé par vissage dans le passage interne (3) du corps de prise (2), au moins une partie du passage interne (3) du corps de prise (2) comprenant un taraudage (30) interne complémentaire du filetage (92) porté par la paroi périphérique externe (93) du corps de cartouche (90).

3. Ventilateur selon l'une des revendications précédentes, **caractérisé en ce que** le corps de cartouche (90) à filtre (9) comprend en outre une extrémité avant (94) conformée pour pouvoir être entrainée en rotation par un outil de vissage, lors du vissage ou du dévissage de la cartouche à filtre (9) au sein du passage interne (3) du corps de prise (2).

4. Ventilateur selon la revendication 2, **caractérisé en ce que** le taraudage (30) est aménagé dans une région d'entrée (31) du passage (3) du corps de prise (2).

5. Ventilateur selon la revendication 3, **caractérisé en ce que** l'extrémité avant (94) du corps de cartouche (90) comprend une empreinte (95) pour un outil de vissage/dévissage.

6. Ventilateur selon la revendication 5, **caractérisé en ce que** l'empreinte (95) est de forme polygonale, de préférence une forme hexagonale pour clé à 6 pans.

7. Ventilateur selon la revendication 1, **caractérisé en ce que** l'élément de filtration (8) est un filtre fritté en bronze ou un filtre tissé monté sur un support en polymère.

8. Ventilateur selon l'une des revendications 1 ou 7, **caractérisé en ce que** l'élément de filtration (8) comprend un filtre de forme conique.

9. Ventilateur selon l'une des revendications 1, 7 ou 8, **caractérisé en ce que** l'élément de filtration (8) comprend un filtre fritté présentant des pores » de diamètres compris entre 60 et 100 µm environ.

10. Ventilateur selon la revendication 1, **caractérisé en ce que** la prise de raccordement (1) est choisie parmi les prises de type NIST ou DISS.

11. Ventilateur selon la revendication 1, **caractérisé en ce que** le circuit de gaz interne du ventilateur (10) est par ailleurs relié fluidiquement à une micro-soufflante.

12. Ventilateur selon l'une des revendications précédentes, **caractérisé en ce qu'**il comprend une carcasse externe portant la prise de raccordement (1).

13. Ensemble de ventilation d'un patient (10, 20, 40) comprenant :
- un ventilateur médical (10) comprenant une prise de raccordement (1) selon l'une des revendications précédentes,
- une source de gaz (20) raccordée fluidiquement à la prise de raccordement (1) du ventilateur médical (10), et
- une interface respiratoire patient (40) reliée fluidiquement à une sortie de gaz (14) du ventilateur médical (10) par l'intermédiaire d'un tuyau d'amenée de gaz (41) flexible.

14. Ensemble de ventilation selon la revendication 13, **caractérisé en ce que** la source de gaz (20) est une source d'oxygène.

## Patentansprüche

1. Medizinisches Beatmungsgerät (10), welches einen inneren Gaskreislauf (12) umfasst, der mit einem an dem medizinischen Beatmungsgerät (10) angeordneten Verbindungsanschluss (1) in Fluidverbindung steht, wobei der Verbindungsanschluss (1) umfasst:
- einen Anschlusskörper (2), der axial von einem inneren Durchgang (3) durchquert wird, der eine Einlassöffnung (4), die von einem freien proximalen Ende (1a) des Anschlusskörpers (2) getragen wird, mit einer Auslassöffnung (5) verbindet, und Mittel zur Steuerung des Flusses (6, 7) umfasst, um die Strömung des Gases innerhalb des inneren Durchgangs (3) zu steuern, die in dem inneren Durchgang (3) zwischen der Einlass- (4) und der Auslassöffnung (5) angeordnet sind, wobei die Mittel zur Steuerung des Flusses (6, 7) ein bewegliches Ventil (6) umfassen, das mit einem Ventilsitz (7) zusammenwirkt, und
- ein Filterelement (8), das in dem inneren Durchgang (3) des Anschlusskörpers (2) angeordnet ist, wobei das Filterelement (8) herausziehbar innerhalb des inneren Durchgangs (3) des Anschlusskörpers (2) angebracht ist, wobei das Einsetzen oder Herausziehen des Filterelements (8) über die Einlassöffnung (4) des inneren Durchgangs (3) erfolgt, die von dem freien proximalen Ende (1a) des Anschlusskörpers (2) getragen wird, wobei das Filterelement (8) in einer Filterpatrone (9) angeordnet ist, die einen Patronenkörper (90) umfasst, der axial von einem inneren Kanal (91) durchquert wird, der in dem inneren Kanal (91) angeordnet ist,
**dadurch gekennzeichnet, dass**:
- das bewegliche Ventil (6) aus Silikon besteht und eine Regenschirmform aufweist, die von einer Stange gebildet wird, die von einer flachen oder leicht gewölbten Scheibe überragt wird, und
- das Filterelement (8) ein Sinterfilter oder Gewebefilter ist.

2. Beatmungsgerät nach Anspruch 1, **dadurch gekennzeichnet, dass** der Patronenkörper (90) der Filterpatrone (9) ein Gewinde (92) umfasst, das in seiner äußeren Umfangswand (93) angeordnet ist, um durch Einschrauben in dem inneren Durchgang (3) des Anschlusskörpers (2) befestigt zu werden, wobei wenigstens ein Teil des inneren Durchgangs (3) des Anschlusskörpers (2) ein Innengewinde (30) umfasst, das zu dem von der äußeren Umfangswand (93) des Patronenkörpers (90) getragenen Gewinde (92) komplementär ist.

3. Beatmungsgerät nach einem der vorhergehenden Ansprüche, **dadurch gekennzeichnet, dass** der Patronenkörper (90) der Filterpatrone (9) außerdem ein vorderes Ende (94) umfasst, das dafür ausgebildet ist, beim Einschrauben oder Losschrauben der Filterpatrone (9) innerhalb des inneren Durchgangs (3) des Anschlusskörpers (2) von einem Schraubwerkzeug drehend angetrieben werden zu können.

4. Beatmungsgerät nach Anspruch 2, **dadurch gekennzeichnet, dass** das Innengewinde (30) in einem Eingangsbereich (31) des Durchgangs (3) des Anschlusskörpers (2) angeordnet ist.

5. Beatmungsgerät nach Anspruch 3, **dadurch gekennzeichnet, dass** das vordere Ende (94) des Patronenkörpers (90) eine Einprägung (95) für ein Einschraub-/Lösewerkzeug umfasst.

6. Beatmungsgerät nach Anspruch 5, **dadurch gekennzeichnet, dass** die Einprägung (95) von polygonaler Form ist, vorzugsweise von sechseckiger Form für einen Sechskantschlüssel.

7. Beatmungsgerät nach Anspruch 1, **dadurch gekennzeichnet, dass** das Filterelement (8) ein Sinterfilter aus Bronze oder ein auf einer Halterung aus Polymer angebrachtes Gewebefilter ist.

8. Beatmungsgerät nach einem der Ansprüche 1 oder 7, **dadurch gekennzeichnet, dass** das Filterelement (8) ein kegelförmiges Filter umfasst.

9. Beatmungsgerät nach einem der Ansprüche 1, 7 oder 8, **dadurch gekennzeichnet, dass** das Filterelement (8) ein Sinterfilter umfasst, das Poren mit Durchmessern zwischen ungefähr 60 und 100 µm aufweist.

10. Beatmungsgerät nach Anspruch 1, **dadurch gekennzeichnet, dass** der Verbindungsanschluss (1) aus den Anschlüssen vom Typ NIST oder DISS ausgewählt ist.

11. Beatmungsgerät nach Anspruch 1, **dadurch gekennzeichnet, dass** der innere Gaskreislauf des Beatmungsgerätes (10) außerdem mit einem Mikrogebläse in Fluidverbindung steht.

12. Beatmungsgerät nach einem der vorhergehenden Ansprüche, **dadurch gekennzeichnet, dass** es ein Außengehäuse umfasst, das den Verbindungsanschluss (1) trägt.

13. Beatmungsanordnung (10, 20, 40) für einen Patienten, welche umfasst:
- ein medizinisches Beatmungsgerät (10), das einen Verbindungsanschluss (1) umfasst, nach einem der vorhergehenden Ansprüche,
eine Gasquelle (20), die mit dem Verbindungsanschluss (1) des medizinischen Beatmungsgerätes (10) in Fluidverbindung steht, und
- eine Patientenbeatmungsschnittstelle (40), die über einen Gaszuführungsschlauch (41) mit einem Gasauslass (14) des medizinischen Beatmungsgerätes (10) in Fluidverbindung steht.

14. Beatmungsanordnung nach Anspruch 13, **dadurch gekennzeichnet, dass** die Gasquelle (20) eine Sauerstoffquelle ist.

## Claims

1. Medical ventilator (10) comprising an internal gas circuit (12) in fluidic communication with a connection socket (1) arranged on said medical ventilator (10), said connection socket (1) comprising:
- a socket body (2) traversed axially by an internal passage (3) connecting an inlet orifice (4), carried by a free proximal end (1a) of the socket body (2), to an outlet orifice (5), and comprising flow control means (6, 7) which control the circulation of the gas within said internal passage (3) and are arranged in the internal passage (3) between the inlet orifice (4) and outlet orifice (5), said flow control means (6, 7) comprising a movable valve (6) cooperating with a valve seat (7), and
- a filtration element (8) arranged in the internal passage (3) of the socket body (2), the filtration element (8) being mounted in a removable fashion within the internal passage (3) of the socket body (2), the insertion or removal of said filtration element (8) taking place through the inlet orifice (4) of the internal passage (3) carried by the free proximal end (1a) of the socket body (2), said filtration element (8) being arranged in a cartridge with filter (9), comprising a cartridge body (90) traversed axially by an internal duct (91), arranged in the internal duct (91),
**characterized in that**:
- the movable valve (6) is made of silicone and has an umbrella shape formed by a rod surmounted by a flat or slightly curved disc, and
- the filtration element (8) is a fritted or woven filter.

2. Ventilator according to Claim 1, **characterized in that** the cartridge body (90) with filter (9) comprises a thread (92) arranged in its outer peripheral wall (93) in order to be fixed by screwing in the internal passage (3) of the socket body (2), at least part of the internal passage (3) of the socket body (2) comprising an internal thread (30) complementary to the thread (92) carried by the outer peripheral wall (93) of the cartridge body (90) .

3. Ventilator according to either of the preceding claims, **characterized in that** the cartridge body (90) with filter (9) further comprises a front end (94) shaped so as to be able to be driven in rotation by a screwing tool, when screwing or unscrewing the filter cartridge (9) within the internal passage (3) of the socket body (2) .

4. Ventilator according to Claim 2, **characterized in that** the internal thread (30) is arranged in an inlet region (31) of the passage (3) of the socket body (2).

5. Ventilator according to Claim 3, **characterized in that** the front end (94) of the cartridge body (90) comprises a recess (95) for a screwing/unscrewing tool.

6. Ventilator according to Claim 5, **characterized in that** the recess (95) has a polygonal shape, preferably a hexagonal shape for a hexagonal wrench.

7. Ventilator according to Claim 1, **characterized in that** the filtration element (8) is a sintered bronze filter or a woven filter mounted on a polymer support.

8. Ventilator according to either of Claims 1 and 7, **characterized in that** the filtration element (8) comprises a filter of conical shape.

9. Ventilator according to one of Claims 1, 7 and 8, **characterized in that** the filtration element (8) comprises a sintered filter having pores with diameters of between 60 and 100 µm approximately.

10. Ventilator according to Claim 1, **characterized in that** the connection socket (1) is chosen from sockets of the NIST or DISS type.

11. Ventilator according to Claim 1, **characterized in that** the internal gas circuit of the ventilator (10) is moreover fluidically connected to a micro-blower.

12. Ventilator according to one of the preceding claims, **characterized in that** it comprises an external casing carrying the connection socket (1).

13. Ventilation assembly (10, 20, 40) for ventilating a patient, comprising:
- a medical ventilator (10) comprising a connection socket (1) according to one of the preceding claims,
- a gas source (20) fluidically connected to the connection socket (1) of the medical ventilator (10), and
- a patient breathing interface (40) fluidically connected to a gas outlet (14) of the medical ventilator (10) by way of a flexible gas supply pipe (41).

14. Ventilation assembly according to Claim 13, **characterized in that** gas source (20) is an oxygen source.
